# EUROPÄISCHE PATENTANMELDUNG

(11) **EP 3 944 839 A1**
(43) Veröffentlichungstag der Anmeldung: **02.02.2022**
(21) Anmeldenummer: 21183493.2
(22) Anmeldetag: 02.07.2021
(51) Int. Cl.: A61F 5/10, A61F 5/30

(54) **DREIPUNKT-RHIZARTHROSEEXTENSIONSORTHESE AUS KUNSTSTOFF ZUR EFFEKTIVEN BEHANDLUNG VON ARTHROSEN UND ARTHRIDEN DES DAUMENSATTELGELENKES**

(30) Priorität: 30.07.2020 DE 202020104418 U
(71) Anmelder: Seidel, Herbert Erich, 49777 Klein Berssen (DE)
(72) Erfinder: Seidel, Herbert Erich, 49777 Klein Berssen (DE)
(74) Vertreter: Jabbusch, Matthias

(57) **Zusammenfassung**

Bei einer Dreipunkt-Rhizarthroseextensionsorthese aus thermoplastischem Kunststoff oder carbonfaserverstärktem Kunststoff nach Abdruck einer vorherigen angepassten Rhizarthroseextensionsorthese oder nach Print zur effektiven konservativen Behandlung von Arthrosen und Arthritiden des Daumensattelgelenkes mit einer halbmondförmigen Pelotte aus PE oder Silikon zum minimalen Auseinanderdrücken des Daumensattelgelenkes, ist vorgesehen, dass sie zumindest ein Verschlussmittel hat, das nach Anlage der Orthese an einer Hand über dem Mittelhandrücken verlaufend anordbar ist.

## Beschreibung

Die Erfindung betrifft eine Dreipunkt-Rhizarthroseextensionsorthese aus thermoplastischem Kunststoff zur effektiven konservativen Behandlung von Arthrosen und Arthritiden des Daumensattelgelenkes mit einer halbmondförmigen Pelotte mit Extensionssehnenkanal aus PE oder Silikon zum minimalen Auseinanderdrücken des arthrotischen Daumensattelgelenkes.

Eine Dreipunkt-Rhizarthroseextensionsorthese kann auch aus einem carbonfaserverstärktem Kunststoff (CFK) nach Abdruck von einer vorher angewendeten Dreipunkt-Rhizarthroseextensionsorthese oder eine nach digitalem Scann geprinteten Dreipunkt-Rhizarthroseextensionsorthese gefertigt werden, zur effektiven konservativen Behandlung von Arthrosen und Arthritiden des Daumensattelgelenkes mit einer halbmondförmigen Pelotte mit Extensionssehnenkanal aus PE oder Silikon zum minimalen Auseinanderdrücken des arthrotischen Daumensattelgelenkes.

Die Rhizarthrose und Rhizarthritis ist insbesondere bei Frauen nach dem Klimaterium eine dem rheumatischen Formenkreis zuzurechnende, schmerzhafte Erkrankung des Daumensattelgelenkes. Bei dieser Erkrankung luxiert das Daumensattelgelenk und die Gelenkknorpel und die Gelenkinnenhäute werden in vier Stadien progressiv zerstört. Die medizinzische Therapie erfolgt je nach Stadium durch Medikation, Kortisoninjektionen, Bandagen und Orthesen sowie operative Eingriffe. Die Rhizarthrose beeinträchtigt nicht nur nachhaltig die Lebensqualität, sondern auch die Arbeitsfähigkeit der Patienten, bis hin zum Verlust des Arbeitsplatzes.

In der DE 20 2012 009 952 U1 ist bereits eine gattungsgemäße Rhizarthroseextensionsorthese beschrieben worden. Diese Rhizarthroseextensionsorthese ist aus einem engmaschig perforiertem, auch möglicherweise gepolstertem, Niedertemperaturthermoplast auf einem Gipsmodell des Patienten oder eine Fertigungsform hergestellt. Sie besteht aus einem die Mittelhand und den Daumen einschließlich des Daumenendgelenkes zirkulär umfassenden Orthesenkörper, der im Mittelhandbereich volar eine Öffnungsweite von etwa 1 cm bis 2 cm hat. Die Rhizarthroseextensionsorthese wird durch ein aufgenietetes oder geklebtes Klett-Flauschband verschlossen. Bei der bekannten Orthese verläuft dieses Klett-Flauschband volar, also in der Innenhand bei angelegter Orthese.

Der bekannte Rhizarthroseextensionsorthesenrohling wird mit ihrem Körper in einem Wasserband auf die notwendige Temperatur erwärmt, an die Mittelhand und den Daumen des Patienten und dann unter zirkulärem Zug angeformt. Im Bereich des proximalen, arthrotischen Daumensattelgelenkes wird eine flügelförmige anatomisch gestaltete Pelotte aus weichem PU-Schaum (PPT oder ähnlich) oder Silikon unter einer rinnenförmigen Freilegung der Extensionssehne des Daumens gefertigten Pelotte unter leichtem distalen Druck eingeklebt. In der Nachsorge wird eine weitere Pelotte in den lateralen Bereich des Orthesenkörpers eingeklebt. Die Orthese kann dauerhaft bei dauerhaft bei Rhizarthrosen der Stadien 2 bis 4 eingesetzt werden, soweit keine operative Lösung bevorzugt wird.

Schon mit der aus der DE 20 2012 009 952 U1 bekannten Orthese wurde erreicht, dass das subluxierte oder luxierte Sattelgelenk durch die individuelle Anpassung des Orthesenkörpers in die anatomisch korrekte Form gebracht wurde und durch die eingeklebte anatomisch geformte Extensionspelotte minimal auseinandergedrückt wird. Es kam dadurch bei den meisten Patientinnen zu einer intraartikulären Druckentlastung und damit zu einer Reduzierung bzw. dem völligen Abklingen des Daumenschmerzens.

Problem war jedoch die Verschließung der Orthese auf der Volarseite und die damit verbundenen hygienischen Probleme an der Innenhand.

Der Erfindung liegt daher die Aufgabe zugrunde, die bekannte Orthese weiter zu verbessern.

Diese Aufgabe ist erfindungsgemäß dadurch gelöst, dass sie zumindest ein Verschlussmittel hat, das nach Anlage der Orthese an einer Hand auf dem Handrücken verlaufend anordbar ist.

Bei der vorliegenden Orthese wird also das Verschlussmittel von der volaren auf die dorsale Seite verlegt. Der Verschluss erfolgt bei anliegender Orthese auf dem Handrücken, dadurch wird die Hand entlastet und die durch die Orthese erreichte Spreizung des Daumensattelgelenkes unterstützt. Das hygienische Problem der verstärkten Schweißbildung und Verschmutzung in der Innenhand wird durch die Verbindungsspange (b) zwischen der Mittelhanddaumenfassung und der gepolsterten Zeigefingerfassung (c) und der gepolsterten Mittelhandkleinfingeranlage (b) aus perforiertem Thermoplast auf der Innenhandseite gelöst. Es ergibt sich eine intensivere Dreipunktentspannung auf die Hand des Patienten. Die Gelenkfläche des Mittelhandknochens des Daumens wird durch das Dreipunktsystem im Daumensattelgelenk wirkungsvoller von der Gelenkfläche des großen Vieleckbeines getrennt (F1), dadurch ergibt sich eine bessere Schmerzfreiheit beziehungsweise Schmerzreduktion. Der Daumen wird vom Zeigefinger weggezogen, dadurch ergibt sich eine Schmerzfreiheit.

Nach einer ersten Weiterbildung der Erfindung ist vorgesehen, dass der Druck auf die Daumenspitze beim Greifen über die definiert angebrachte Extensionspelotte proximal des Sattelgelenkes über die drei Druckflächen des zirkulären Orthesenkorpus auf die Mittelhand ableitbar ist.

Bei einer Orthese nach dieser Weiterbildung ist der Druck auf die Daumenspitze beim Greifen, insbesondere bei einem Pinzettengriff und auch bei einem Rundgriff, auf die über die Extensionspelotte auf dem zirkulären aus Niedertemperaturthermoplast bestehendem Orthesenkorpus auf die Mittelhand im Bereich des Mittelhandknochens des Zeigefingers (F3) und des lateralen Mittelhandknochens des Kleinfingers (F2) abgeleitet. Durch die neue Dreipunkt-Hebelwirkung wird die Extension des Sattelgelenkes verstärkt und somit die Druck- und Schmerzfreiheit verbessert.

Das auf dem Handrücken verlaufend anordbare Verschlussmittel ist vorzugsweise ein Riemen, der die nötigen Kräfte aufnehmen kann. Das Verschlussmittel kann mit einem Schließorgan versehen sein, dieses Schließorgan ist insbesondere ein Klettabschnitt. Ein Klettabschnitt ist auf einfache Weise zu öffnen oder zu schließen.

Nach einer anderen Weiterbildung der Erfindung ist vorgesehen, dass für das über den Mittelhandrücken verlaufende Verschlussmittel ein Unterstützungselement vorgesehen ist. Mit einem derartigen Unterstützungselement wird der Verlauf des Verschlussmittels auf dem Handrücken stabilisiert. Das Unterstützungselement verläuft dabei selbst über den Handrücken, es überspannt vorzugsweise die Mittelhandknochen vom kleinen bis zum Mittelfinger.

Das Unterstützungselement ist dabei vorzugsweise eine Pelotte, die an den Kunststoff der Orthese, insbesondere an einen Kunststoffkörper angesetzt ist. Die Mittelhandknochen werden also mit einer Pelotte überspannt, diese ist vorzugsweise gepolstert.

Nach einer nächsten Weiterbildung kann vorgesehen sein, dass die halbmondförmige Pelotte bei Anlage der Dreipunkt-Rhizarthroseextensionsorthese an einer Hand bis über den Mittelhandknochen des Zeigefingers reicht. Während die das Unterstützungselement ausbildende Pelotte die Mittelhandknochen vom kleinen bis zum mittleren Finger überspannt, ist ein Gegenstück durch die halbmondförmige Pelotte gegeben. Diese ist verbreitert, sodass sie bis über den Mittelhandknochen des Zeigefingers reicht. Das vorgesehene Verschlussmittel überspannt die an den Kunststoffkörper angesetzte Pelotte und die halbmondförmige Pelotte, die beiden vorgesehenen Pelotten unterstützen aber dieses Verschlussmittel gegen ein Abrutschen bzw. Abkippen.

Zudem kann noch vorgesehen sein, dass die halbmondförmige Pelotte als zwei hügelförmige Pelotte ausgebildet ist. Die Pelotte kann eine halbmondförmige Grundform haben, auf der zwei Hügel aufstehen.

Ausführungsbeispiele der Erfindung, aus denen sich weitere erfinderische Merkmale ergeben, sind in der Zeichnung dargestellt. Es zeigen:
- Figuren 1 und 2:: Seitenansichten eines ersten Ausführungsbeispiels einer erfindungsgemäßen Dreipunkt-Rhizarthroseextensionsorthese,
- Figur 3:: eine Seitenansicht der Orthese nach Figuren 1 und 2 nach einem Anlegen an eine menschliche Hand,
- Figur 4:: eine weitere Seitenansicht der Orthese nach Figuren 1 und 2 bei einer Anlage an eine menschliche Hand, und
- Figur 5:: ein zweites Ausführungsbeispiel einer erfindungsgemäßen Dreipunkt-Rhizarthroseextensionsorthese bei einer Anlage an eine menschliche Hand.

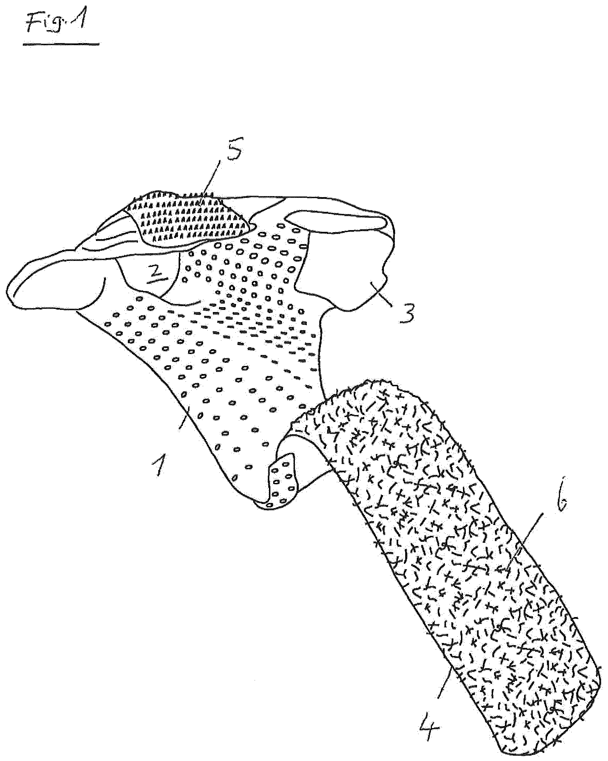

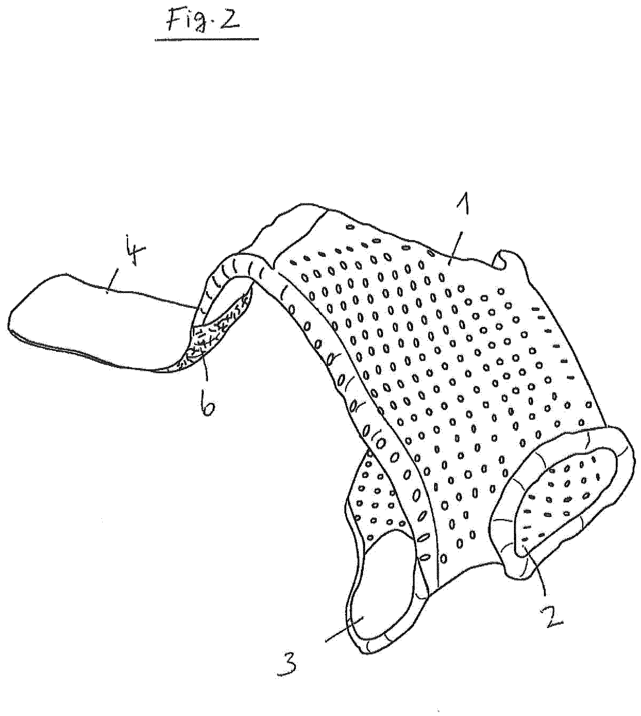

Die Orthese in Figuren 1 und 2 ist aus einem thermoplastischen Kunststoffkörper 1 gefertigt. Der thermoplastische Kunststoffkörper 1 ist so geformt, dass er an die Innenhand angelegt werden kann. Zur Hindurchführung des Daumens ist ein Durchbruch 2 vorgesehen. In den Kunststoffkörper 1 ist zur Abstützung des Daumens eine Pelotte 3 eingebracht.

Die Orthese hat ein Verschlussmittel. Dieses Verschlussmittel besteht aus einem an dem Kunststoffkörper 1 angelängten Riemen 4 sowie aus einem auf dem Kunststoffkörper 1 angebrachten Anlagestück 5. Der Riemen 4 kann umgelegt werden und auf das Anlagestück 5 aufgelegt werden. Der Riemen 4 trägt ein Verschlussmittel, das als Klettverschlussabschnitt 6 ausgebildet ist. Der Klettverschlussabschnitt 6 kann an das Anlagestück 5 angelegt werden, das einen Gegenpart für den Klettverschlussabschnitt 6 ausbildet.

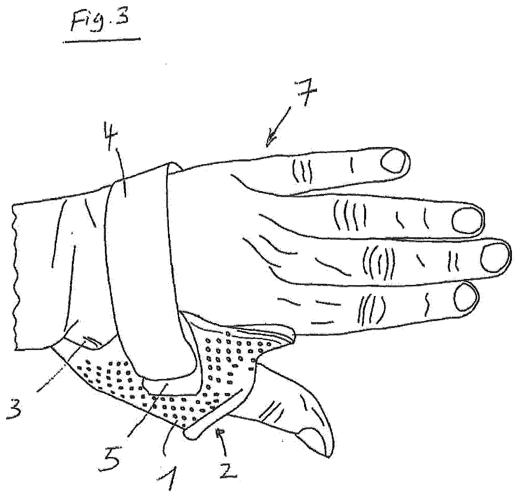

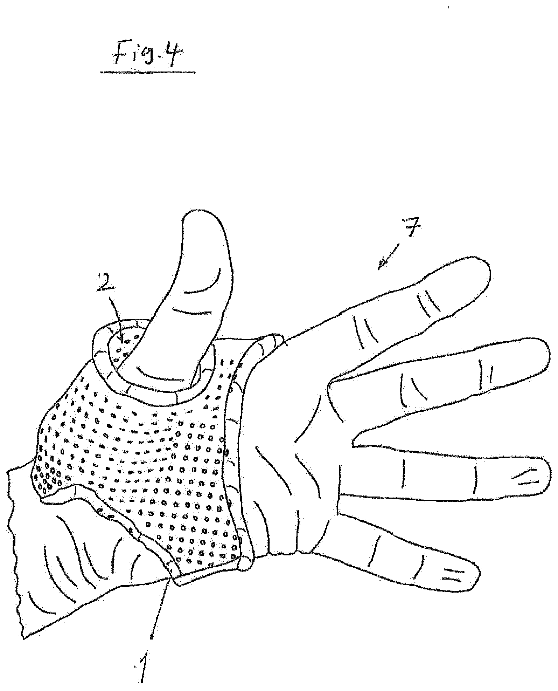

Die Anlage der Orthese an eine menschliche Hand 7 in Figur 3 zeigt den Verschluss der Orthese mit dem Riemen 4, der an das Anlagestück 5 angelegt ist. Das Verschlussmittel läuft auf dem Rücken der Hand 7. Figur 4 zeigt noch einmal den Orthesenkörper aus Kunststoff 1, der die Innenfläche der Hand 7 abdeckt.

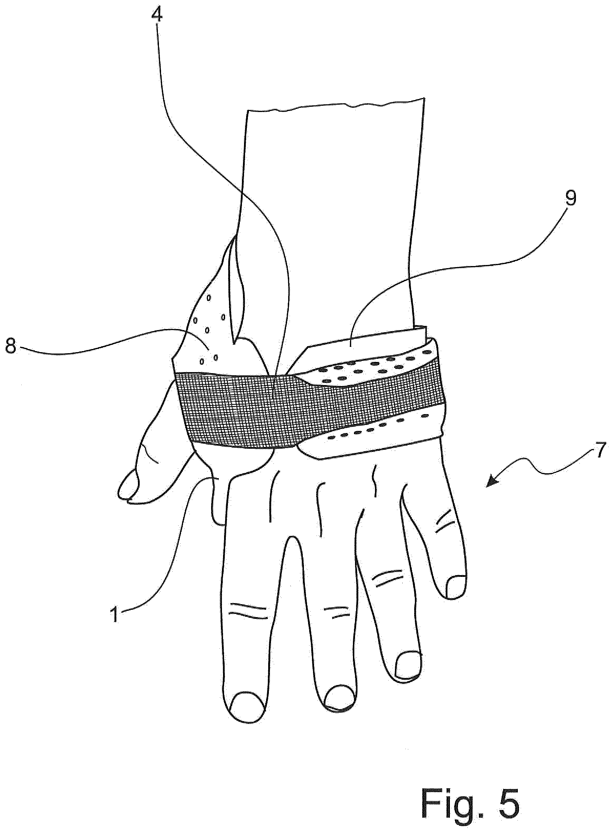

In Figur 5 ist das zweite Ausführungsbeispiel der erfindungsgemäßen Dreipunkt-Rhizarthroseextensionsorthese wieder nach Anlage an eine menschliche Hand 7 dargestellt. Der Kunststoffkörper 1 verläuft im Wesentlichen über die Handfläche der Hand 7. Figur 5 zeigt die halbmondförmige Pelotte 8, die bei diesem Ausführungsbeispiel bis über den Mittelhandknochen des Zeigefingers der Hand 7 reicht. Die Pelotte 8 ist also gegenüber dem Ausführungsbeispielen nach Figuren 1 bis 4 verbreitert.

Die Orthese in Figur 5 weist auch einen Riemen 4 als Verschlussmittel auf. Dieser Riemen 4 ist auf dem Handrücken durch eine Pelotte 9 unterstützt. Diese Pelotte 9 verläuft von der dem Daumen gegenüberliegenden Handkante über den Handrücken, über die Mittelhandknochen von Zeigefinger, Ringfinger und Mittelfinger. Die verbreiterte Pelotte 8 und die Pelotte 9 stoßen auf dem Handrücken nahezu aneinander, sie bilden so einen stabilen Untergrund für das Verschlussmittel mit dem Riemen 4. Der Riemen 4 hat wieder im Bereich der Pelotte 8 ein Verschlussmittel, das wieder als Klettverschlussabschnitt ausgebildet ist. Somit ist auch das Verschlussmittel für den Riemen 4 auf dem Handrücken angeordnet.

## Patentansprüche

1. Dreipunkt-Rhizarthroseextensionsorthese aus thermoplastischem Kunststoff oder carbonfaserverstärktem Kunststoff nach Abdruck einer vorherigen angepassten Rhizarthroseextensionsorthese oder nach Print zur effektiven konservativen Behandlung von Arthrosen und Arthritiden des Daumensattelgelenkes mit einer halbmondförmigen Pelotte aus PE oder Silikon zum minimalen Auseinanderdrücken des Daumensattelgelenkes,
**dadurch gekennzeichnet,**
**dass** sie zumindest ein Verschlussmittel hat, das nach Anlage der Orthese an einer Hand (7) über dem Mittelhandrücken verlaufend anordbar ist.

2. Dreipunkt-Rhizarthroseextensionsorthese nach Anspruch 1, **dadurch gekennzeichnet, dass** der Druck auf die Daumenspitze beim Greifen über die definiert angebrachte Extensionspelotte proximal des Sattelgelenkes über die drei Druckflächen des zirkulären Orthesenkorpus auf die Mittelhand ableitbar ist.

3. Dreipunkt-Rhizarthroseextensionsorthese nach Anspruch 1 oder 2, **dadurch gekennzeichnet, dass** das Verschlussmittel ein Riemen (4) ist.

4. Dreipunkt-Rhizarthroseextensionsorthese nach einem der vorhergehenden Ansprüche, **dadurch gekennzeichnet, dass** dem Verschlussmittel ein Schließorgan zugeordnet ist.

5. Dreipunkt-Rhizarthroseextensionsorthese nach Anspruch 4, **dadurch gekennzeichnet, dass** das Schließorgan ein Klettverschlussabschnitt (6) ist.

6. Dreipunkt-Rhizarthroseextensionsorthese nach einem der vorhergehenden Ansprüche, **dadurch gekennzeichnet, dass** für das Verschlussmittel ein Unterstützungselement vorgesehen ist.

7. Dreipunkt-Rhizarthroseextensionsorthese nach Anspruch 6, **dadurch gekennzeichnet, dass** das Unterstützungselement eine an den Kunststoff, insbesondere an einen Kunststoffkörper (1) angesetzte Pelotte (9) ist.

8. Dreipunkt-Rhizarthroseextensionsorthese nach einem der vorgehenden Ansprüche, **dadurch gekennzeichnet, dass** die halbmondförmige Pelotte (8) bei Anlage der Dreipunkt-Rhizarthroseextensionsorthese an eine Hand bis über den Mittelhandknochen des Zeigefingers reicht.

9. Dreipunkt-Rhizarthroseextensionsorthese nach einem der vorhergehenden Ansprüche, **dadurch gekennzeichnet, dass** die halbmondförmige Pelotte (8) als zwei hügelförmige Pelotte ausgebildet ist.
